# EUROPEAN PATENT APPLICATION

(11) **EP 4 186 531 A1**
(43) Date of publication of application: **31.05.2023**
(21) Application number: 22198968.4
(22) Date of filing: 30.09.2022
(51) Int. Cl.: A61L 9/20

(54) **INDUSTRIAL PURIFIER OF THE TYPE WITH IMPROVED ELECTRONIC CONTROL AND MANAGEMENT**

(30) Priority: 30.11.2021 IT 202100030212
(71) Applicant: Losma S.p.a., 24035 Curno (IT)
(72) Inventor: PADOVAN, Luca Emilio, 24020 PRADALUNGA BG (IT)
(74) Representative: Modiano, Micaela Nadia

(57) **Abstract**

An industrial purifier (1) of the type with improved electronic control and management, comprising a hollow body (5) which is delimited laterally by at least one grille-like surface (6) and comprising on the inside light-emitting means (7) adapted to emit ultraviolet light for air purification and electronic control and management means (9) associated with the light-emitting means (7); purifier further comprising means for verifying the correct operation of the light-emitting means (7), means (10) for signaling the state of operation of the light-emitting means (7), and means for automatically regulating the supply power of the light-emitting means (7) as a function of their state and of the light-emitting capacity required.

## Description

The present invention relates to an industrial purifier of the type with improved electronic control and management.

In the sector of disinfecting environments the use is known of ultraviolet light for air purification.

In more detail, this operation occurs by virtue of the fact that, from research carried out, ultraviolet light appears to be capable of at least partially reducing the viral load present in the air.

Thus industrial purifiers have been provided that are capable of emitting ultraviolet light for disinfecting environments.

These purifiers consist substantially of a form of lamp that emits ultraviolet rays, which is provided with a battery of ultraviolet LEDs that are controlled by dedicated electronics.

Such conventional purifiers are not devoid of drawbacks, among which is the fact that in the event of malfunctions there is no immediate feedback on the operation status of the device.

The aim of the present invention consists in providing an industrial purifier of the type with improved electronic control and management in order to be able to monitor its operation status by an operator in real time.

Within this aim, an object of the present invention consists in providing an industrial purifier that is provided with means capable of signaling any malfunctions promptly, indicating the exact location of the malfunction to the operator.

Another object of the present invention consists in providing an industrial purifier that is capable of offering the widest guarantees of reliability and safety in use.

Another object of the present invention consists in providing an industrial purifier that can be provided with technologies that are known per se and which therefore is low-cost.

This aim and these and other objects which will become more apparent hereinafter are achieved by an industrial purifier of the type with improved electronic control and management, comprising a hollow body which is delimited laterally by at least one grille-like surface and comprising on the inside light-emitting means adapted to emit ultraviolet light for air purification and electronic control and management means associated with said light-emitting means, characterized in that it comprises means for verifying the correct operation of said light-emitting means, means for signaling the state of operation of said light-emitting means, and means for automatically regulating the supply power of said light-emitting means as a function of their state and of the light-emitting capacity required.

Further characteristics and advantages of the invention will become more apparent from the detailed description of a preferred, but not exclusive, embodiment of an industrial purifier of the type with improved electronic control and management, which is illustrated for the purposes of non-limiting example in the accompanying drawings wherein:

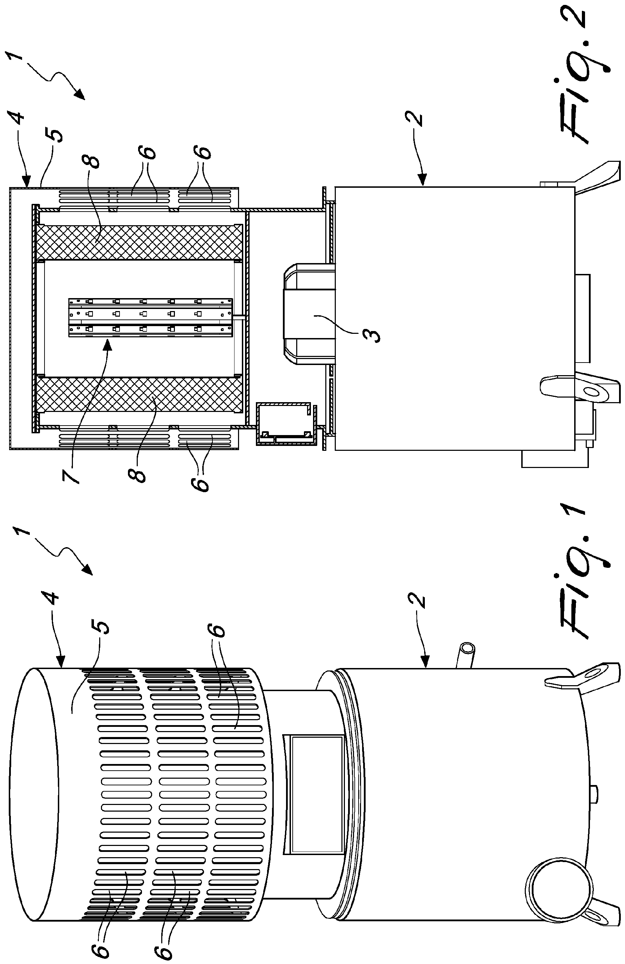

- Figure 1 is a front elevation view of the industrial purifier according to the present invention;
- Figure 2 is a front elevation view of the industrial purifier shown in Figure 1 and partially open;
- Figure 3 is a perspective view of the light-emitting means of the industrial purifier shown in the previous figures;
- Figure 4 is a perspective view seen from above of the electronic control and management means of the industrial purifier shown in the previous figures;
- Figure 5 is a perspective view seen from below of the electronic control and management means of the industrial purifier shown in the previous figures;
- Figure 6 is a front elevation view of the means for signaling the operation of the industrial purifier shown in the previous figures.

With reference to the figures, the industrial purifier of the type with improved electronic control and management, generally designated by the reference numeral 1, comprises a main structure which defines a first section 2 which accommodates aspirator means 3 adapted to aspirate the air present in the surrounding environment and adapted to direct it into a second section 4 which is directly connected to the first section 2.

In more detail, the second section 4 is defined by a hollow body 5 which is delimited laterally by at least one grille-like surface 6 and which comprises on the inside light-emitting means 7 adapted to emit ultraviolet light for air purification.

In this way, the aspirator means 3 direct the air in the hollow body 5 directly onto the light-emitting means 7 which are surrounded by filtering means 8 interposed between these and the grille-like surface 7 in order to filter the air propelled by the aspirator means 3.

The light-emitting means 7 are associated with electronic control and management means 9 which, according to the invention, are provided with means for verifying the correct operation of the light-emitting means 7, means for signaling 10 the state of operation of the light-emitting means 7, and means for automatically regulating the supply power of the light-emitting means 7 as a function of their state and of the light-emitting capacity required.

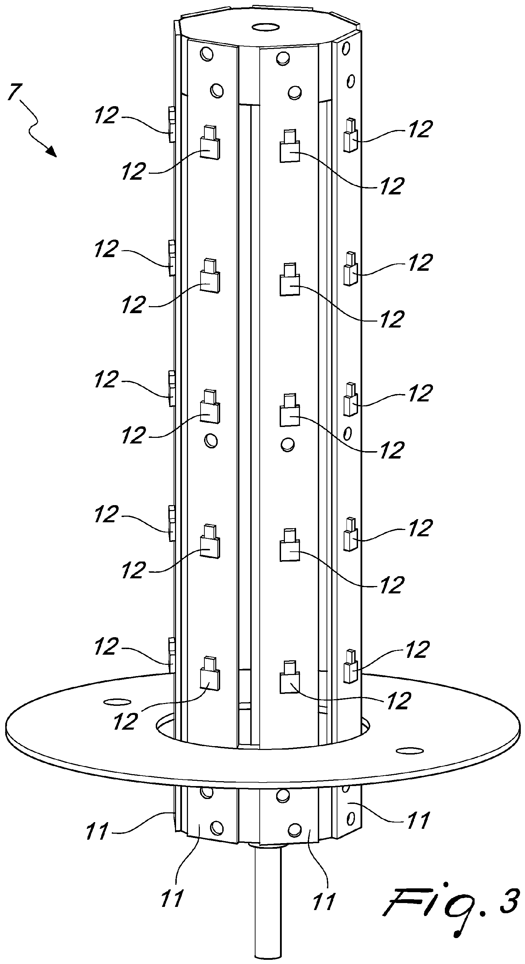

In more detail, with particular reference to Figure 3, the light-emitting modules 7 comprise a plurality of light-emitting modules 11 arranged according to a 360° cylindrical array and each one constituted, in turn, by a battery of ultraviolet LEDs 12, abbreviated to UV-C, which is constituted by a PCB (printed circuit board) of the METALCORE type of dimensions, for example, 270x20 millimeters and 1.6 millimeters thick.

On each individual light-emitting module 11 therefore five ultraviolet LEDs 12 (Vishay model VLMU35CT2-275-120 UV-C LEDs or, alternatively, Seoul model CUD8AF4D UV-C LEDs) are installed in parallel.

The electronic control and management means 9 comprise one or both of a main electronic board 13, called the master, and a secondary electronic board 14, called the slave, which are functionally mutually connected in parallel over an RS232 interface with 3-volt logic levels, by a three-wire multipolar cable.

In more detail, the electronic control and management means 9 can manage a maximum number of ten light-emitting modules 11 with a single electronic board 13 or 14 or, in particular applications, they can manage a maximum number of twenty light-emitting modules 11 by connecting the two electronic boards 13 and 14 in parallel with one board set in master mode while the other is in slave mode.

In this way, each light-emitting module 11 can be connected to the control card by a six-pole multipolar cable with Molex Pico-SPOX series connectors in such a manner as to be powered with a voltage, for example, of approximately 6.5 volts with a constant current absorption of 0.85 amperes.

During normal operation, each light-emitting module 11 will be struck by a flow of pre-treated air which will ensure an optimal thermal dissipation.

The power supply of the electronic board 13 and/or 14 can be direct current with a voltage of 24 volts with a maximum consumption of approximately 67 watts (equal to approximately 2.8 amperes) with a load constituted by ten installed light-emitting modules 11. By connecting the two boards in parallel (master-slave), the consumption will be equal to approximately 134 watts.

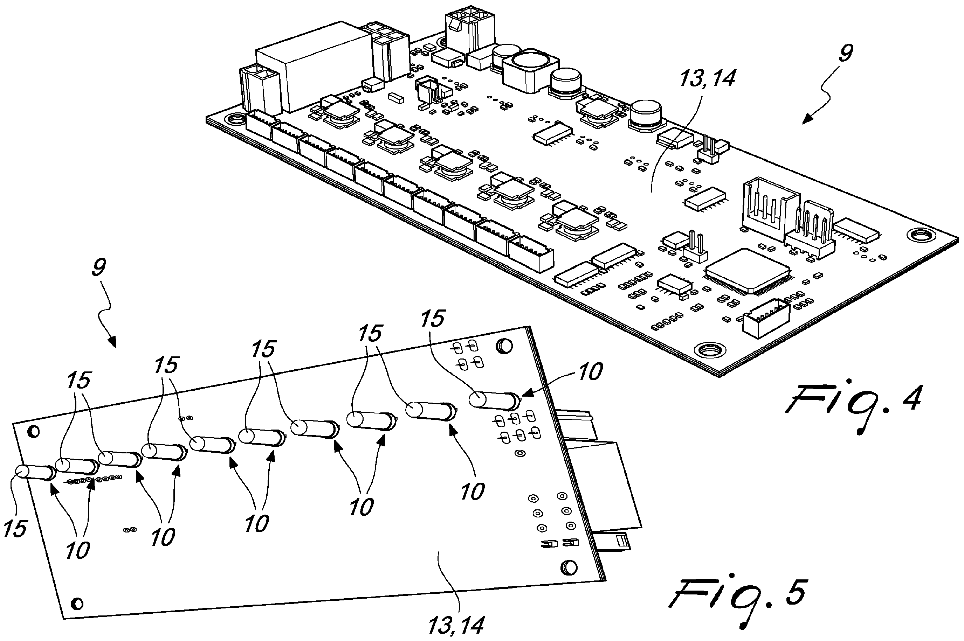

Advantageously, the electronic components of the at least one electronic board 13 or 14, which can have dimensions of 180x84 millimeters and a thickness of 1.6 millimeters and has four holes for affixing to the purifier structure 1, as shown in Figure 4, are all arranged on one side of the board, and the means 10 for signaling comprise a plurality of multicolor signaling LEDs 15, equal in number to the number of light-emitting modules 11, so as to signal continuously the state of operation thereof, signaling with a first color, for example green, their correct operation and signaling with a second color, for example red, a malfunction thereof.

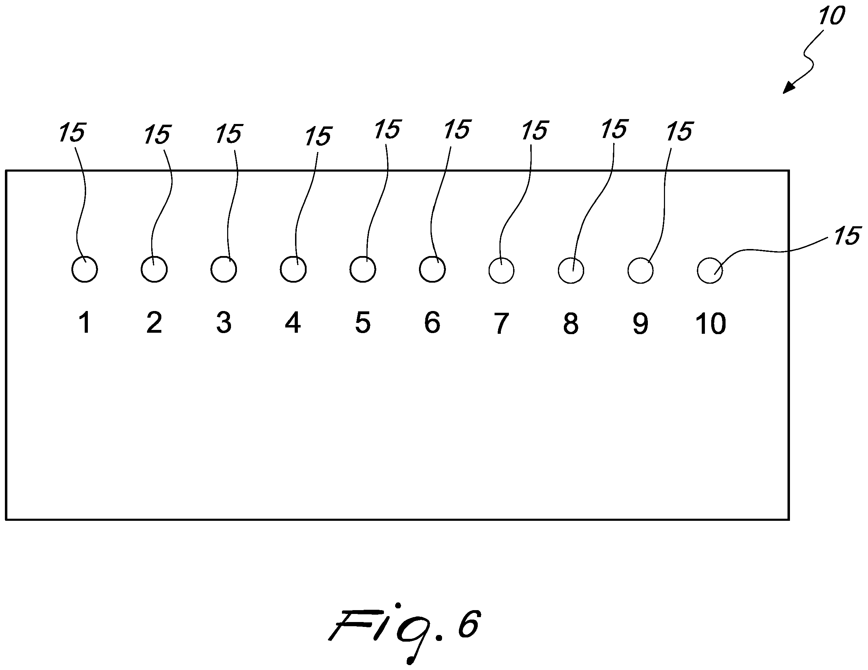

Conveniently, the means 10 for signaling which are arranged on the opposite side from the electronic components mentioned above, are covered by a mask, shown in Figure 6, which has a plurality of holes at the multicolor signaling LEDs 15.

In more detail, the electronic board 13 or 14 has a powerful microprocessor that accurately controls the entire sterilization cycle and verifies the operation status of each single light-emitting module 11, signaling malfunctions and the state of wear of each ultraviolet LED 12.

In particular, the electronic board 13 or 14 is equipped with five direct current power supplies which operate in STEPDOWN mode; each power supply is capable of driving up to two light-emitting modules 11.

The electronic board 13 or 14 automatically regulates the output current depending on whether one light-emitting module 11 or two light-emitting modules 11 are connected to that specific power supply (850 milliamperes or 425 milliamperes).

The modulation of the power occurs using PWM (pulse-width modulation) logic, which makes it possible to optimize the disinfection process and to reduce the consumption of electricity, thus defining the abovementioned means for automatically regulating the supply power.

The connections on the control card are listed below:
- 24 volt DC power supply connector: Molex Mini-Fit Jr., four poles, vertical, product code 39281043;
- Connectors for connecting UV-C LED modules: there are ten Pico-SPOX connectors, six poles, vertical, product code 874370643 (one connector for each light-emitting module 11);

- Connector for connecting power-on button: Pico-SPOX, two poles, vertical, product code 874370243;
- Connector for in-out connection: Molex Mini-Fit Jr., six poles, vertical, product code 39281063. Specifically, two contacts will normally be mutually closed using a safety switch. If the safety switch is opened, the power to the current drivers of the light-emitting modules 11 will be cut and then this anomaly will be reported to the microprocessor which will produce a specific alarm signal. It is possible to install an emergency button in series with the safety switch. On the other contacts, there is the power supply of the blue LED strip (24 volts DC) and optionally the power supply of the slave board (24 volts DC);
- 24 volt DC slave power supply connector: Molex Mini-Fit Jr., two poles, vertical, product code 39281023;
- Connector for connecting slave board: Pico-SPOX, six poles, vertical, product code 874370643, in order to connect another control card if twenty light-emitting modules 11 are necessary;
- JTAG connector: 4 poles, TE code TE 280371-2, for firmware updates by way of an ST-LINK external programmer;
- Serial connector for debugging/configuration: four poles, Molex code 22272041.

Advantageously, the electronic board 13 or 14 comprises a jumper which can be set selectively in closed mode, for its automatic powering-on and subsequently for starting the sterilization cycle (in this mode the power-on button is needed only for resetting any alarms), or in open mode, for its powering-on or powering-off by means of a power button.

More specifically, a short press of the button resets any alarms, while a long press powers down the electronic board.

The electronic board 13 or 14 in master-slave mode will be able to manage a maximum of twenty light-emitting modules 11.

By way of a four-pole ON/OFF DIP-SWITCH, it is possible to set the number of modules installed.

Via the DIP-SWITCH setting, the control card is capable of knowing how many light-emitting modules 11 are installed and therefore, upon being powered on, it automatically executes a check of the operation status (voltage and current reading) of each individual ultraviolet LED 12 of each individual light-emitting module 11.

In the event of an anomaly with a single ultraviolet LED 12, the electronic board 13 or 14 will signal the error by switching on the multicolor signaling LEDs 15 with the red color of the specific light-emitting module 11.

If no anomalies are reported, the electronic board 13 or 14 will use the green color of the multicolor signaling LEDs 15 to signal the correct operation of the individual light-emitting module 11.

If the light-emitting module 11 is not installed, the corresponding multicolor signaling LEDs 15 will remain switched off.

Below is a brief legend for reading the multicolor signaling LEDs 15:
- GREEN LED: correct operation of n^{th} light-emitting module 11;
- RED LED: anomaly in n^{th} light-emitting module 11, or n^{th} light-emitting module 11 has failed (in both cases it is necessary to replace the light-emitting module 11);
- LED OFF: n^{th} light-emitting module 11 is not installed;
- FLASHING GREEN LED: n^{th} light-emitting module 11 is approaching failure (operating margin of approximately 500 hours). The electronic board 13 or 14 counts the hours of operation of the individual light-emitting modules 11 and reports when the maximum operating life is reached;
- GREEN+ORANGE LED (alternately flashing): emergency button activated or the safety switch is not activated (the switch is open);
- RED+ORANGE LED (alternately flashing): the electricity supply does not conform to the specifications of the control card (24 volts DC and 2.8 amps for an absorbed power of 67 watts).

In practice it has been found that the industrial purifier achieves the intended aim and objects.

The industrial purifier, thus conceived, is susceptible of numerous modifications and variations, all of which are within the scope of the appended claims.

Moreover, all the details may be substituted by other, technically equivalent elements.

In practice the materials employed, provided they are compatible with the specific use, and the contingent dimensions and shapes, may be any according to requirements and to the state of the art.

The disclosures in Italian Patent Application No. 102021000030212 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An industrial purifier (1) of the type with improved electronic control and management, comprising a hollow body (5) which is delimited laterally by at least one grille-like surface (6) and comprising on the inside light-emitting means (7) adapted to emit ultraviolet light for air purification and electronic control and management means (9) associated with said light-emitting means (7), **characterized in that** it comprises means for verifying the correct operation of said light-emitting means (7), means for signaling the state of operation of said light-emitting means (7), and means for automatically regulating the supply power of said light-emitting means (7) as a function of their state and of the light-emitting capacity required.

2. The industrial purifier (1) according to claim 1, **characterized in that** said light-emitting means (7) comprise a plurality of light-emitting modules (11) arranged according to a 360° cylindrical array.

3. The industrial purifier (1) according to claim 2, **characterized in that** each one of said light-emitting modules (11) comprises a battery of ultraviolet LEDs (12).

4. The industrial purifier (1) according to one or more of the preceding claims, **characterized in that** said electronic control and management means (9) comprise either or both of a main electronic board (13) and a secondary electronic board (14) which can be functionally mutually connected in parallel.

5. The industrial purifier (1) according to one or more of the preceding claims, **characterized in that** the electronic components of said at least one electronic board (13, 14) are all arranged on one side of said at least one electronic board (13, 14) and **in that** said means (10) for signaling comprise a plurality of multicolor signaling LEDs (15), equal in number to the number of said light-emitting modules (11), so as to signal continuously the state of operation of said light-emitting modules (11), signaling correct operation with a first color and signaling a malfunction of said light-emitting modules (11) with a second color.

6. The industrial purifier (1) according to one or more of the preceding claims, **characterized in that** said at least one electronic board (13, 14) comprises a jumper which can be set selectively in closed mode, for its automatic powering-on and subsequently for starting the sterilization cycle, or in open mode, for its powering-on or powering-off by means of a power button.

7. The industrial purifier (1) according to one or more of the preceding claims, **characterized in that** it comprises aspirator means (3) adapted to aspirate the air present in the surrounding environment and adapted to direct it into said hollow body (5) directly onto said light-emitting means (7).

8. The industrial purifier (1) according to one or more of the preceding claims, **characterized in that** it comprises filtering means (8) interposed between said light-emitting means (7) and said grille-like surface (6) in order to filter the air propelled by said aspirator means (3).
